# EUROPEAN PATENT APPLICATION

(11) **EP 4 159 221 A1**
(43) Date of publication of application: **05.04.2023**
(21) Application number: 21818712.8
(22) Date of filing: 01.06.2021
(51) Int. Cl.: A61K 33/00, A61K 8/19, A61P 17/00, A61Q 17/04, A61Q 19/00

(54) **ULTRAVIOLET LIGHT SENSITIVITY REDUCING AGENT**

(30) Priority: 02.06.2020 JP 2020096165
(71) Applicant: Kao Corporation, Chuo-ku Tokyo 103-8210 (JP)
(72) Inventor: SAYAMA, Keimon, Haga-gun, Tochigi 321-3497 (JP); YUKI, Katsuyuki, Tokyo 131-8501 (JP); ISHIKAWA, Junko, Haga-gun, Tochigi 321-3497 (JP)
(74) Representative: Vossius & Partner Patentanwälte Rechtsanwälte mbB
(86) International application number: PCT/JP2021/020780
(87) International publication number: WO 2021/246385

(57) **Abstract**

Provided is an ultraviolet light sensitivity reducing agent for reducing the sensitivity of the skin to ultraviolet light. An ultraviolet light sensitivity reducing agent for skin, comprising carbon dioxide gas as an active ingredient.

## Description

### Field of the Invention

The present invention relates to an ultraviolet light sensitivity reducing agent for skin.

### Background of the Invention

Solar ultraviolet light reaching the surface of the earth is classified into long-wavelength ultraviolet light UV-A (from 320 to 400 nm) and medium-wavelength ultraviolet light UV-B (from 290 to 320 nm), and UV-B reaching the upper dermis of human skin causes an erythematous reaction (sunburn) in the skin. Ultraviolet erythema is observed several hours after UV-B exposure, peaks at about 24 hours, and then disappears. After disappearance of erythema, delayed tanning (suntan) occurs. Reactive oxygen species (ROS) generated in the skin by ultraviolet light and biooxides generated by the reaction with ROS are considered to be involved in the formation of ultraviolet erythema.

The state of erythemaous reaction by UV-B varies greatly depending on the sensitivity of the skin to ultraviolet light (UV-B). The minimum ultraviolet dose which causes erythema is expressed as minimal erythema dose (MED), and the lower the MED is, that is, the higher sensitive the skin to ultraviolet light is, the smaller the ultraviolet dose which causes erythema on the skin is. Therefore, it is important to reduce the sensitivity of the skin to ultraviolet light in order to protect the skin from ultraviolet light.

On the other hand, a blood circulation promoting action is generally known as a physiological action of carbon dioxide gas, and cosmetics, bathing agents, and the like utilizing the blood circulation promoting action of carbon dioxide gas have been proposed (Patent Literature 1).

It has also been reported that application of a foamable external preparation for skin brings about effects such as moist feeling and elasticity of the skin, brightening of the skin, and confirmation of redness of the skin (Patent Literature 2), that a carbon dioxide external preparation is effective for treatment of acne with redness, thinning of parts of the face, and the like (Patent Literature 3), and that a viscous composition containing carbon dioxide is effective for itching associated with tinea pedis and atopic dermatitis (Patent Literature 4).

However, it is not known that carbon dioxide gas has an effect of reducing the ultraviolet light sensitivity of the skin.

(Patent Literature 1) JP-A-2011-93877
(Patent Literature 2) JP-A-2018-199731
(Patent Literature 3) JP-A-2017-2073
(Patent Literature 4) JP-A-2010-275322

### Summary of the Invention

The present invention relates to the following 1) to 10) :
1) An ultraviolet light sensitivity reducing agent for skin, comprising carbon dioxide gas as an active ingredient.
2) An ultraviolet erythema formation inhibitor for skin, comprising carbon dioxide gas as an active ingredient.
3) Use of carbon dioxide gas for producing an ultraviolet light sensitivity reducing agent for skin.
4) Use of carbon dioxide gas for producing an ultraviolet erythema formation inhibitor for skin.
5) Carbon dioxide gas for use in reducing ultraviolet light sensitivity of skin.
6) Carbon dioxide gas for use in inhibiting ultraviolet erythema formation of skin.
7) Non-therapeutic use of carbon dioxide gas for reducing ultraviolet light sensitivity of skin.
8) Non-therapeutic use of carbon dioxide gas for inhibiting ultraviolet erythema formation of skin.
9) A method for reducing ultraviolet light sensitivity of skin, comprising applying carbon dioxide gas to skin of a subject in need thereof.
10) A method for inhibiting ultraviolet erythema formation of skin, comprising applying carbon dioxide gas to skin of a subject in need thereof.

### Brief Description of Drawings

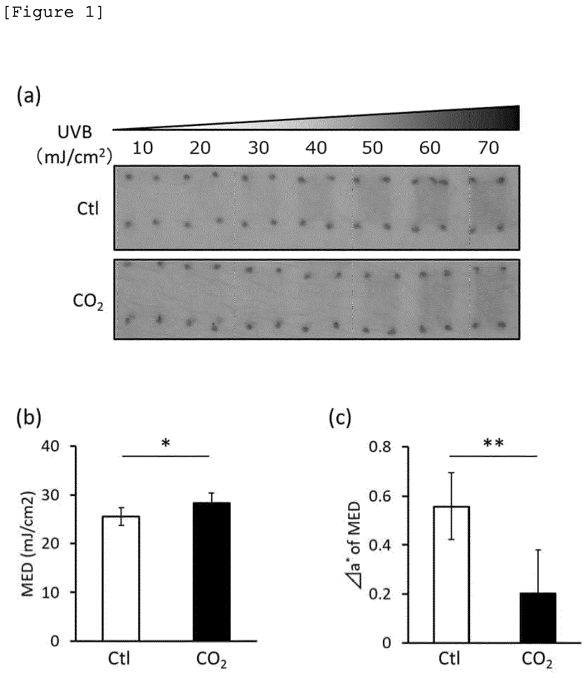

Figure 1(a) is a diagram showing erythema formation at a UV-B irradiation site. Figure 1(b) is a diagram showing the effect on MED by application of a carbon dioxide gas-containing cream. Figure 1(c) is a diagram showing the amount of change in skin color (a* value) at the UV-B irradiation site.

### Detailed Description of the Invention

The present invention relates to a provision of an ultraviolet light sensitivity reducing agent for reducing the sensitivity of the skin to ultraviolet light.

The present inventors found that the sensitivity of the skin to ultraviolet light can be reduced in a case where carbon dioxide gas is applied to the skin.

According to the present invention, by applying carbon dioxide gas to the skin, it is possible to reduce the sensitivity of the skin to ultraviolet light and to inhibit the formation of erythema due to ultraviolet light.

As shown in Examples below, in a case where a composition containing carbon dioxide gas is applied to human skin, the minimal erythema dose (MED) at the application site is significantly increased. Inhibition of erythema formation due to UV-B is observed at the application site, and the amount of change in skin color (a* value: redness) is significantly reduced. As described above, the MED is an indicator of ultraviolet light sensitivity.

Therefore, carbon dioxide gas can serve as an ultraviolet light sensitivity reducing agent for skin or an ultraviolet erythema formation inhibitor for skin (hereinafter also referred to as "ultraviolet light sensitivity reducing agent for skin or the like"), and by supplying carbon dioxide gas to the skin of human or the like, the sensitivity of the skin to ultraviolet light can be reduced and the formation of ultraviolet erythema can be inhibited.

Carbon dioxide gas can be used for reducing ultraviolet light sensitivity of skin, for inhibiting the formation of ultraviolet erythema, and for producing an ultraviolet light sensitivity reducing agent for skin or the like.

Here, the use of carbon dioxide gas for humans may be therapeutic use or non-therapeutic use. The "non-therapeutic" means not including a medical treatment; namely, not including a surgery, therapy or diagnosis method for a human, more specifically, a concept not including a surgery, therapy or diagnosis method practiced on a human by a doctor, a health care worker, or a person under a doctor's supervision. In the present invention, examples of the non-therapeutic use include use of carbon dioxide gas, and the like for cosmetic or aesthetic purposes.

In the present invention, the "ultraviolet light sensitivity" refers to the degree of inflammatory reactions such as erythema to be induced by ultraviolet light, mainly medium-wavelength ultraviolet light UV-B (from 290 to 320 nm), indicated by the intensity or the duration of the inflammatory reactions, and the degree of the inflammatory reactions means the sensitivity to ultraviolet light.

Further, "reducing the ultraviolet light sensitivity" means to mitigate or suppress the sensitivity to ultraviolet light, to increase the minimal erythema dose (MED), to suppress the development of the inflammatory reactions such as erythema to be induced by ultraviolet light, and to reduce the amount of change in the redness of skin color (a* value), which is an indicator of inflammatory reactions.

The means of ultraviolet light sensitivity reducing agent for skin or the like of the present invention is not particularly limited as long as they can supply carbon dioxide gas to the stratum corneum of the skin, and examples thereof include external preparations for skin such as aerosol cosmetics in which carbon dioxide gas is enclosed as a propellant (JP-A-2014-129306, JP-A-2017-125003, and the like), foamable external preparations for skin containing a carbon dioxide gas generating agent which reacts with moisture to generate carbon dioxide gas, nonwoven fabric sheets (JP-A-2015-105451), bathing agents (JP-A-H9-2942, JP-A-2000-191429, and the like), and pads (JP-A-2006-249025, and the like).

In a case where carbon dioxide gas is enclosed as a propellant in an external preparation for skin, the carbon dioxide gas is preferably contained in an amount of 90% by mass or more, more preferably in an amount of 95% by mass or more, further more preferably in an amount of 98% by mass or more, and even more preferably in an amount of 100% by mass based on the total amount of the propellant from the viewpoint of obtaining the effect of reducing ultraviolet light sensitivity of the present invention on the skin.

In the case of preparing an aerosol preparation in which carbon dioxide gas is enclosed as a propellant, the mass ratio of the external preparation for skin (stock solution) and the propellant containing carbon dioxide gas is preferably from 94:6 to 99.5:0.5, more preferably from 95:5 to 99:1, and further more preferably from 96.5:3.5 to 98.5:1.5. The spray form of the aerosol preparation is preferably a foam type in which the external preparation for skin is discharged in the form of foam, from the viewpoint of continuously retaining carbon dioxide gas mixed as a propellant on the skin.

The external preparation for skin to be used as a stock solution may contain various components usually used in external preparation for skin such as cosmetics, quasi-drugs, and pharmaceuticals. Specifically, it is preferable to contain components such as powder, oil, surfactant, water-soluble thickener, and water from the viewpoint of discharging the external preparation for skin in the form of foam, improving the spreading of the foam on the skin, providing excellent use impression, and maintaining the foam to enhance the effect of carbon dioxide gas.

Examples of the powder include one or more powders selected from the group consisting of silicon oxide, titanium oxide, (meth)acrylic acid or a salt thereof/alkyl(meth)acrylate crosspolymer, and silicone elastomer, which have an average particle size from 0.01 to 30 µm.

The (meth)acrylic acid or a salt thereof/alkyl(meth)acrylate crosspolymer is a crosslinked (meth)acrylate-based resin powder obtained by copolymerizing at least one monomer selected from the group consisting of (meth)acrylic acids and salts thereof and at least one monomer selected from the group consisting of alkyl(meth)acrylates. Here, (meth)acrylic acid is a generic term for acrylic acid and methacrylic acid, and alkyl(meth)acrylate is a generic term for alkyl acrylate and alkyl methacrylate. Specific examples thereof include lauryl methacrylate/ethylene glycol dimethacrylate/sodium methacrylate copolymer.

The silicone elastomer is a generic term of silicone having a crosslinked structure obtained by polymerization of a monomer and a derivative thereof. For example, silicones and derivatives thereof obtained by polymerizing or copolymerizing a monomer selected from the group consisting of dimethicone, vinyl dimethicone, phenyl vinyl dimethicone, lauryl dimethicone, lauryl polydimethylsiloxyethyl dimethicone and the like are preferable, (dimethicone/vinyl dimethicone) crosspolymer, (dimethicone/phenyl vinyl dimethicone) crosspolymer, (vinyl dimethicone/lauryl dimethicone) crosspolymer, (vinyl dimethicone/methicone silsesquioxane) crosspolymer, and (PEG-15/lauryl dimethicone) crosspolymer are more preferable, (dimethicone/vinyl dimethicone) crosspolymer, (vinyl dimethicone/lauryl dimethicone) crosspolymer and (PEG-15/lauryl dimethicone) crosspolymer are further more preferable, and (dimethicone/vinyl dimethicone) crosspolymer is even more preferable.

Examples of the (dimethicone/vinyl dimethicone) crosspolymer include commercially available products such as: those in the form of solid, for example, TREFIL E-506S (active ingredient: 100% by mass) (manufactured by Dow Corning Toray Co., Ltd.); mixtures with a liquid oil, for example, KSG-15 (active ingredient: 7% by mass) as a mixture with decamethylcyclopentasiloxane (KF-995), KSG-16 (active ingredient: 25% by mass) as a mixture with low-viscosity dimethylpolysiloxane (KF-96A-6cs) (manufactured by Shin-Etsu Chemical Co., Ltd.), TREFIL E-508 (active ingredient: 70% by mass) as a mixture with dimethicone; and mixtures with water such as BY29-119 (active ingredient: 63% by mass) and BY29-129 (active ingredient: 63% by mass). Examples of the (dimethicone/phenyl vinyl dimethicone) crosspolymer include commercially available products such as: mixtures with a liquid oil, for example, KSG-18A (active ingredient: 15% by mass) as a mixture with diphenylsiloxyphenyl trimethicone (KF-56A) (manufactured by Shin-Etsu Chemical Co., Ltd.).

The content of the powder in the stock solution is preferably 0.1% by mass or more, more preferably 0.5% by mass or more, further more preferably 0.8% by mass or more, and preferably 2.5% by mass or less, more preferably 2.0% by mass or less, further preferably 1.5% by mass or less in the stock solution of the present invention, from the viewpoint of improving the uniform applicability of the foam containing carbon dioxide gas to the skin and the permeability of carbon dioxide gas into the skin. A specific content range is preferably from 0.1 to 2.5% by mass, more preferably from 0.5 to 2.0% by mass, and further more preferably from 0.8 to 1.5% by mass.

The oil is not limited as long as it is used for ordinary cosmetics, and examples thereof include hydrocarbon oils, silicone oils, ester oils, ether oils, fluorinated oil, and the like. More specific examples thereof include linear or branched hydrocarbon oils such as light isoparaffin, liquid paraffin, liquid isoparaffin, squalane, and squalene; silicone oils such as dimethylpolysiloxane, cyclomethicone, dimethicone, trisiloxanemethyltrimethicone, ethyltrisiloxane, dimethylcyclopolysiloxane, methylphenylpolysiloxane, methylhydrogenpolysiloxane, and higher alcohol-modified organopolysiloxane; monoester oils such as isononyl isononanoate, isotridecyl isononanoate, and alkyl benzoate (C12-15) which is an ester of benzoic acid and an aliphatic alcohol having 12 to 15 carbon atoms; diester oils such as neopentyl glycol dicaprate; triester oils such as caprylic/capric triglyceride and triglyceryl 2-ethylhexanoate; ether oils such as alkyl-1,3-dimethylbutyl ether, dicaprylyl ether, and dicaprylyl ether; and fluorinated oils such as fluoropolyether and perfluoroalkylether silicone, and from the viewpoint of improving the spreading of the foam, one or more selected from the group consisting of hydrocarbon oils, ester oils, and silicone oils are preferable, and hydrocarbon oils, monoester oils, triester oils, and silicone oils are more preferable, and monoester oil and silicone oil are preferable.

The viscosity of the oil is 1 mPa·s or more, preferably 2 mPa·s or more, more preferably 3 mPa·s or more, further more preferably 4 mPa.s or more, and is 100 mPa·s or less, preferably 50 mPa·s or less, more preferably 30 mPa·s or less, further more preferably 20 mPa·s or less, from the viewpoint of improving the permeability of carbon dioxide gas into the skin. The oil has a viscosity of from 1 to 100 mPa·s, preferably from 2 to 50 mPa·s, more preferably from 3 to 30 mPa·s, and further more preferably from 4 to 20 mPa·s.

Here, the viscosity is measured at 25°C with a BM viscometer (manufactured by Toki Sangyo Co., Ltd.) (rotor No. 1, 60 rpm, 1 minute).

One or more oils can be used, and the content thereof is 1% by mass or more, preferably 2% by mass or more, more preferably 4% by mass or more, and 25% by mass or less, preferably 15% by mass or less, more preferably 12% by mass or less in the stock solution from the viewpoint of improving the permeability of carbon dioxide gas into the skin and the feeling of skin coating after application. The content of the oil in the stock solution is from 1 to 25% by mass, preferably from 2 to 15% by mass, and more preferably from 4 to 12% by mass.

Examples of the surfactant include a nonionic surfactant, an anionic surfactant, a cationic surfactant, and an amphoteric surfactant, and the like, and in the present invention, a nonionic surfactant is preferably used from the viewpoint of the amount of carbon dioxide gas dissolved in the stock solution, promotion of solubility, and foaming properties during application. The nonionic surfactant used in the present invention is a nonionic surfactant having a HLB of from 3 to 20, and the HLB of the nonionic surfactant is preferably 7 or more, more preferably 9 or more, and further more preferably 11 or more, from the viewpoint of improving the permeability of foam into the skin and the storage stability of the composition, and preferably 18 or less, more preferably 16 or less, and further more preferably 15 or less, from the viewpoint of improving the feeling of skin coating after application. The HLB of the nonionic surfactant is preferably from 7 to 18, more preferably from 9 to 16, and further more preferably from 11 to 15.

Here, HLB (Hydrophilic-Lipophilic Balance) indicates the molecular weight of the hydrophilic group portion in the total molecular weight of the surfactant, and is obtained by Griffin's equation. The HLB of a mixed surfactant containing two or more nonionic surfactants can be determined by calculating the arithmetic mean of the HLB values of the nonionic surfactants based on their blending ratios.

Specific examples of the nonionic surfactant include sorbitan fatty acid ester, glycerol fatty acid ester, polyoxyethylene fatty acid ester, polyoxyethylene sorbitan fatty acid ester, polyoxyethylene alkyl ether, and polyoxyethylene hydrogenated castor oil, and the like, and sorbitan fatty acid ester, glycerol fatty acid ester, polyoxyethylene sorbitan fatty acid ester, and polyoxyethylene hydrogenated castor oil are preferable, sorbitan fatty acid ester and polyoxyethylene hydrogenated castor oil are more preferable, and polyoxyethylene hydrogenated castor oil is further more preferable.

In the polyoxyethylene hydrogenated castor oil, the average number of moles of ethylene oxide added is preferably from 20 to 90, more preferably from 30 to 80, and further more preferably from 50 to 70, from the viewpoint of improving the permeability of carbon dioxide gas into the skin and stability of foam.

The nonionic surfactant can be used alone or in combination of two or more, and the content thereof is 0.05% by mass or more, preferably 0.1% by mass or more, more preferably 0.3% by mass or more, and 6% by mass or less, preferably 2% by mass or less, more preferably 1% by mass or less in the stock solution from the viewpoint of improving the spreading of the foam, the permeability of carbon dioxide gas into the skin, and the feeling of skin coating after application. The content of the nonionic surfactant in the stock solution is from 0.05 to 6% by mass, preferably from 0.1 to 2% by mass, and more preferably from 0.3 to 1% by mass.

The water-soluble thickener may be any one used for ordinary cosmetics, and examples thereof include carrageenan, dextrin, methyl cellulose, ethyl cellulose, hydroxyethyl cellulose, hydroxypropyl cellulose, polyvinyl alcohol, polyacrylic acid, polymethacrylic acid, carboxyvinyl polymer, acrylic acid/alkyl methacrylate copolymer, xanthan gum, carboxymethyl chitin, chitosan, and the like. These have the effect of enhancing the viscosity of the stock solution, inhibiting rapid occurrence of the foam, and improving the stability. Furthermore, carboxyvinyl polymer and acrylic acid/alkyl methacrylate copolymer are preferable, and acrylic acid/alkyl methacrylate copolymer is more preferable, from the viewpoint of improving the permeability of carbon dioxide gas into the skin and the feeling of skin coating after application. Here, the acrylic acid/alkyl methacrylate copolymer is a copolymer of C10-30 alkyl acrylate and acrylic acid, methacrylic acid or a lower alkyl ester thereof and is crosslinked with an allyl ether of sucrose or an allyl ether of pentaerythritol, and a commercially available product, such as Pemulen TR-1, Pemulen TR-2, Carbopol ETD 2020, Carbopol 1342, Carbopol 1382 (all manufactured by Lubrizol Advanced Materials, Inc) can be used.

The water-soluble thickener can be used alone or in combination of two or more selected from the above, and the content thereof is 0.1% by mass or more, preferably 0.15% by mass or more, more preferably 0.2% by mass or more, and 1% by mass or less, preferably 0.8% by mass or less, more preferably 0.5% by mass or less in the stock solution. The content of the water-soluble thickener in the stock solution is from 0.1 to 1% by mass, preferably from 0.15 to 0.8% by mass, and more preferably from 0.2 to 0.5% by mass.

Water acts as a solvent, and the content thereof is preferably 55% by mass or more, more preferably 65% by mass or more, further more preferably 75% by mass or more, and is preferably 95% by mass or less, more preferably 93% by mass or less, and further more preferably 90% by mass or less in the stock solution. The content of water is preferably from 55 to 95% by mass, more preferably from 65 to 93% by mass, and further more preferably from 75 to 90% by mass in the stock solution.

The viscosity of the stock solution at 25°C is preferably 500 mPa.s or more, more preferably 1,000 mPa·s or more, further more preferably 1,500 mPa·s or more, and preferably 20,000 mPa.s or less, more preferably 10,000 mPa·s or less, further more preferably 7,000 mPa·s or less from the viewpoint of improving the stability, the foam discharge properties, and the permeability of carbon dioxide gas into the skin. The viscosity of the stock solution at 25°C is preferably from 500 to 20,000 mPa.s, more preferably from 1,000 to 10,000 mPa·s, and further more preferably from 1,500 to 7,000 mPa·s.

Here, the viscosity is the value measured at rotor No. 3, 12 rpm, for 1 minute using a BM viscometer (manufactured by Toki Sangyo Co., Ltd.) at 25°C. When the viscosity exceeds 10,000 mPa·s, it is measured at rotor No. 3, 6 rpm, for 1 minute.

The carbon dioxide gas generating agent used in the foamable external preparation for skin usually includes a preparation which contains a carbon dioxide gas generating substance and an acidic substance and which reacts with water to generate carbon dioxide gas.

Here, the acidic substance may be either an inorganic acid or an organic acid, and one or more thereof are used. Examples of the organic acid include one or more selected from the group consisting of succinic acid, fumaric acid, malic acid, adipic acid, tartaric acid, benzoic acid, citric acid, pyrrolidone carboxylic acid, and salicylic acid. Specific examples of the inorganic acid include one or more selected from the group consisting of phosphoric acid, boric acid, metasilicic acid, and silicic anhydride. Among them, from the viewpoint of securing the amount of carbon dioxide gas generated, an organic acid is preferable, one or more selected from the group consisting of citric acid, malic acid, fumaric acid, succinic acid, and tartaric acid are more preferable, and one or more selected from the group consisting of citric acid, malic acid, and fumaric acid are further more preferable.

Examples of the carbon dioxide gas generating substance include carbonates, and specific examples thereof include one or more selected from the group consisting of sodium carbonate, sodium hydrogen carbonate, potassium carbonate, potassium hydrogen carbonate, calcium carbonate, magnesium carbonate, and sodium sesquicarbonate. Among them, the carbonate is preferably one or more selected from the group consisting of sodium carbonate and sodium hydrogen carbonate from the viewpoint of ensuring the amount of carbon dioxide gas generated.

Such a carbon dioxide gas generating agent may contain an acidic substance and a carbon dioxide gas generating substance in the same agent, or may contain them in a plurality of agents separately.

The dosage form of the foamable external preparation for skin may be any of a solid form, a liquid form, a gel form, and the like. In the case of a solid form, it may be a granular form, a fine granular form, or a powder form. The external preparation for skin may be in any form of cosmetics, quasi-drugs, and pharmaceuticals, but is preferably used as cosmetics or quasi-drugs. Specific examples thereof include various forms such as a skin lotion, a milky lotion, a cosmetic gel, a pack material, a hair growth agent, a facial cleanser, a cleansing agent, a shampoo, and a conditioner, and a form in which the foamable external preparation for skin is supported on a sheet-like material may also be used. In use, it is preferable to mix with a water-containing substance on the palm of the hand, in a container, or in a sheet-like material, and foam the mixture for use.

The sheet-like material is not particularly limited as long as it is a material which can support a carbon dioxide gas generating agent (acidic substance and carbon dioxide gas generating substance) in the form of powder and can generate a sufficient amount of carbon dioxide gas in the sheet only by pouring water or hot water to the sheet before application to the skin or after application to the skin, and preferred examples thereof include a nonwoven fabric sheet.

Specific examples thereof include a nonwoven fabric sheet containing (A) a powder selected from the group consisting of organic acids and inorganic acids, (B) a carbonate powder, (C) a substance having a melting point of 50°C or more and 110°C or less and being solid at 25°C (for example, a polymer compound including a polyether such as polyethylene glycol or a cellulose derivative such as hydroxyethyl cellulose; a higher fatty acid such as myristic acid or palmitic acid; a higher alcohol such as cetyl alcohol, hexadecyl alcohol, cetearyl alcohol or stearyl alcohol; or a saccharide such as glucose or maltose), and (D) a fiber.

Examples of the bathing agent include a foamable tablet-type bathing agent containing (a) a solid excipient containing 10% by mass or more of a surfactant and a carbonate in an amount of 2 mass times or more of the surfactant, (b) a carbonate or a hydrogen carbonate, and (c) an organic acid, wherein the content of the component (a) is from 3 to 20% by mass in the bathing agent (JP-A-H9-2942), and a foamable cosmetic containing (A) an acid which is solid at normal temperature or an inorganic substance which exhibits acidity when dissolved in water, (B) a carbonate, and (C) a cooling or warming agent (JP-A-2000-191429).

Examples of the pad include a pad including a base layer having water absorbing properties, a protective layer having gas barrier properties provided so as to cover the base layer, and a carbon dioxide gas generating agent enclosed between the protective layer and the base layer, which can be attached to the skin at the peripheral portion of the protective layer (JP-A-2006-249025).

The ultraviolet light sensitivity reducing agent for skin or the like of the present invention may be applied to the skin by a method such as spraying or coating in accordance with an any application plan, and may be applied once to several times a day. The application period can be appropriately determined, and for example, is preferably 1 day or more, more preferably 7 days or more, and further more preferably 14 days or more.

The subject to which the ultraviolet light sensitivity reducing agent for skin or the like of the present invention is applied is not particularly limited as long as it is necessary, but is preferably a human who is highly sensitive to ultraviolet light and is likely to cause erythema due to ultraviolet light or a human who is frequently exposed to ultraviolet light.

The application site of the ultraviolet light sensitivity reducing agent for skin or the like of the present invention is not particularly limited as long as it is human skin, but preferably is the skin of a site frequently exposed to ultraviolet light. Specific examples thereof include the head, face, forehead, cheek, neck, decollete, upper arm, forearm, back of hand, back, abdomen, leg, thigh, lower leg, instep, and the like, of which the face, forehead, cheek, neck, decollete, upper arm, and forearm are preferable, the face, forehead, cheek, neck, and decollete are more preferable, and the face, forehead, and cheek are further more preferable.

The present invention further discloses the following aspects regarding the above-described embodiments.
<1> An ultraviolet light sensitivity reducing agent for skin, comprising carbon dioxide gas as an active ingredient.
<2> An ultraviolet erythema formation inhibitor for skin, comprising carbon dioxide gas as an active ingredient.
<3> Use of carbon dioxide gas for producing an ultraviolet light sensitivity reducing agent for skin.
<4> Use of carbon dioxide gas for producing an ultraviolet erythema formation inhibitor for skin.
<5> Carbon dioxide gas for use in reducing ultraviolet light sensitivity of skin.
<6> Carbon dioxide gas for use in inhibiting ultraviolet erythema formation of skin.
<7> Non-therapeutic use of carbon dioxide gas for reducing ultraviolet light sensitivity of skin.
<8> Non-therapeutic use of carbon dioxide gas for inhibiting ultraviolet erythema formation of skin.
<9> A method for reducing ultraviolet light sensitivity of skin, comprising applying carbon dioxide gas to skin of a subject in need thereof.
<10> A method for inhibiting ultraviolet erythema formation of skin, comprising applying carbon dioxide gas to skin of a subject in need thereof.
<11> In <1> to <10>, the ultraviolet light is preferably UV-B.
<12> In <1> to <10>, the carbon dioxide gas is preferably applied in a form of an external preparation for skin.
<13> In <12>, the external preparation for skin contains carbon dioxide gas in an amount of preferably 90% by mass or more, more preferably 95% by mass or more, further more preferably 98% by mass or more, even more preferably 100% by mass or more, based on the total amount of a propellant.
<14> In <12> or <13>, a mass ratio of the external preparation for skin (stock solution) and the propellant containing carbon dioxide gas is preferably from 94:6 to 99.5:0.5, more preferably from 95:5 to 99:1, and further more preferably from 96.5:3.5 to 98.5:1.5.
<15> In <12> to <14>, the external preparation for skin preferably contains powder, oil, surfactant, water-soluble thickener, and water.
<16> In <12> to <15>, the external preparation for skin preferably has a viscosity of the stock solution at 25°C of preferably 500 mPa·s or more, more preferably 1,000 mPa·s or more, further more preferably 1,500 mPa·s or more, and is preferably 20,000 mPa s or less, more preferably 10,000 mPa s or less, further more preferably 7,000 mPa s or less, and preferably from 500 to 20,000 mPa s, more preferably from 1,000 to 10,000 mPa s, further more preferably from 1,500 mPa s to 7,000 mPa·s.

### Examples

### Example 1

### (1) Test Participants

Nine healthy men in their 20 to 50s were selected, excluding those who had skin symptoms such as atopic dermatitis, those who had skin problems or excessive sunburn at the test site, those who regularly performed outdoor activities which cause extreme sunburn, those who had taken or applied to the test site pharmaceuticals which had an effect on the skin or cosmetic products for the purpose of beauty, and those who were smokers. The test was conducted in accordance with the Declaration of Helsinki, and candidates were briefed on the conditions and methods of the test and informed consent was obtained in advance.

### (2) Test Item

A carbon dioxide gas-containing cream of formula shown in Table 1 was prepared as follows and used as a test item.

Among the components shown in Table 1, components (1), (2), and (15) were stirred at 60°C, and (10) was mixed and stirred. Then, the remaining components were added, and the mixture was stirred to homogeneity and cooled to 25°C to obtain a stock solution, which was used as "control cream". A pressure-resistant container was filled with the obtained stock solution, sealed, and filled with carbon dioxide gas to obtain a "carbon dioxide gas-containing cream".

**[Table 1]**

| Component | | % by mass |
|---|---|---|
| 1 | (Dimethicone/vinyl dimethicone) crosspolymer ∗1 | 0.5 |
| 2 | Polyoxyethylene hydrogenated castor oil ∗2 | 0.5 |
| 3 | Polyoxyethylene/methylpolysiloxane copolymer ∗3 | 0.5 |
| 4 | Acrylic acid/alkyl methacrylate copolymer ∗4 | 0.35 |
| 5 | Methylpolysiloxane ∗5 | 4.14 |
| 6 | Alkyl benzoate (C12-15) ∗6 | 2.0 |
| 7 | Dipropylene glycol | 5.0 |
| 8 | 1,3-Propanediol | 2.0 |
| 9 | Potassium dihydrogen phosphate | 0.2 |
| 10 | Potassium hydroxide solution (A) ∗7 | 0.5 |
| 11 | Disodium edetate | 0.05 |
| 12 | Lauryl methacrylate/ethylene glycol dimethacrylate/sodium methacrylate copolymer aqueous dispersion | q.s. |
| 13 | Preservative | q.s. |
| 14 | Fragrance | q.s. |
| 15 | Purified water | q.s. |
| Stock solution total | | 100.0 |
| Stock solution: carbon dioxide gas | | 97.6 : 2.4 |

| | | |
|---|---|---|
| *1: Silicone KSG-16 (dimethicone/vinyl dimethicone) crosspolymer /KF-96A-6CS = 25/75, manufactured by Shin-Etsu Chemical Co., Ltd. *2: EMANON CH60K, manufactured by Kao Corporation: HLB14 *3: Silicone SH3775M, manufactured by Dow Corning Toray Co., Ltd.: HLB5 *4: Pemulen TR-1, manufactured by Lubrizol Advanced Materials, Inc. *5: KF-96A-6CS, manufactured by Shin-Etsu Chemical Co., Ltd: viscosity 6 mPa·s *6: FINSOLV-TN, manufactured by Innospec Active Chemicals LLC: viscosity 13 mPa·s *7: Aqueous solution containing 46.0 to 50.0% of potassium hydroxide | | |

### (3) Summary of Test

The test was a double blind parallel test in which carbon dioxide gas-containing cream (COz) and control cream (Control: Ctl) were each applied to the inner part of the left upper arm for two weeks.

Two areas to be applied (5 cm × 15 cm) were set on the inner part of the left upper arm, and about 0.5 g of the carbon dioxide gas-containing cream (CO₂) or the control cream (Ctl) was applied to each applying area twice a day in the morning and in the evening. After two weeks, the cream applied sites were washed, and then each cream applied site was irradiated with ultraviolet light (UV-B), and on the next day, MED determination and skin color measurement were performed to confirm erythema formation.

### (4) Ultraviolet irradiation

Seven UV-B irradiation sites were set for each of the two applied areas on the inner part of the left upper arm, and UV-B was irradiated at a dose of 1 mW/cm² in 7 steps at an interval of 10 mJ/cm² (from 10 to 70 mJ/cm²). A UV-B lamp (GL20SE, SANKYO) was used as the ultraviolet ray source, and the UV-B dose was measured using a DERMARAY UV-METER (DM-UV-M2CV-B, Gigahertz-optik).

### (5) MED determination

On the next day of the ultraviolet irradiation, a person in charge of the test visually observed the UV-B irradiation site and made MED determination. At the same time, the UV-B irradiation site was photographed using a digital camera.

### (6) Skin color measurement

The a* values were measured using a spectrophotometric type color difference meter SE-6000 (NIPPON DENSHOKU INDUSTRIES Co.,Ltd.) before and the next day of the ultraviolet irradiation. Measurements were performed three times per site irradiated with UV-B, and the average value was taken as the measured value.

### (7) Statistical analysis

Paired student's t-test was used for comparison of the results between groups (*p < 0.05, ** p < 0.01). All results were expressed as mean ± standard error.

### (8) Results

To confirm the effect of carbon dioxide gas on ultraviolet erythema formation, carbon dioxide gas-containing cream (CO₂) and control cream (Ctl) were applied to the skin of the inner part of the left upper arm, followed by UV-B irradiation to induce erythema formation. As a result, inhibition of erythema formation by UV-B was observed at the site to which the carbon dioxide gas-containing cream (CO₂) was applied as compared with the site to which the control cream (Ctl) was applied (Figure 1a). At this time, the minimal erythema dose (MED) at the carbon dioxide gas-containing cream (CO₂) applied site was significantly increased as compared with the MED at the control cream (Ctl) applied site (Figure 1b). In addition, the amount of change in a* value (redness) before and after ultraviolet irradiation at the site irradiated with UV-B equivalent to 1 MED of the carbon dioxide gas-containing cream (CO₂) applied site was significantly decreased at the carbon dioxide gas-containing cream (CO₂) applied site as compared with the control cream (Ctl) applied site (Figure 1c). These results indicate that carbon dioxide gas reduces the sensitivity of human skin to UV-B and inhibits the formation of ultraviolet erythema.

## Claims

1. An ultraviolet light sensitivity reducing agent for skin, comprising carbon dioxide gas as an active ingredient.

2. An ultraviolet erythema formation inhibitor for skin, comprising carbon dioxide gas as an active ingredient.

3. The ultraviolet light sensitivity reducing agent for skin according to claim 1 or the ultraviolet erythema formation inhibitor for skin according to claim 2, wherein the ultraviolet light is UV-B.

4. The ultraviolet light sensitivity reducing agent for skin or the ultraviolet erythema formation inhibitor for skin according to any one of claims 1 to 3, which is in a form of an external preparation for skin.

5. Use of carbon dioxide gas for producing an ultraviolet light sensitivity reducing agent for skin.

6. Use of carbon dioxide gas for producing an ultraviolet erythema formation inhibitor for skin.

7. Use according to claim 5 or 6, wherein the ultraviolet light is UV-B.

8. Use according to any one of claims 5 to 7, wherein the ultraviolet light sensitivity reducing agent for skin or the ultraviolet erythema formation inhibitor for skin is in a form of an external preparation for skin.

9. Carbon dioxide gas for use in reducing ultraviolet light sensitivity of skin.

10. Carbon dioxide gas for use in inhibiting ultraviolet erythema formation of skin.

11. The carbon dioxide gas according to claim 9 or 10, wherein the ultraviolet light is UV-B.

12. The carbon dioxide gas according to any one of claims 9 to 11, which is used in a form of an external preparation for skin.

13. Non-therapeutic use of carbon dioxide gas for reducing ultraviolet light sensitivity of skin.

14. Non-therapeutic use of carbon dioxide gas for inhibiting ultraviolet erythema formation of skin.

15. The non-therapeutic use according to claim 13 or 14, wherein the ultraviolet light is UV-B.

16. The non-therapeutic use according to any one of claims 13 to 15, wherein the carbon dioxide gas is used in a form of an external preparation for skin.

17. A method for reducing ultraviolet light sensitivity of skin, comprising applying carbon dioxide gas to skin of a subject in need thereof.

18. A method for inhibiting ultraviolet erythema formation of skin, comprising applying carbon dioxide gas to skin of a subject in need thereof.

19. The method according to claim 17 or 18, wherein the ultraviolet light is UV-B.

20. The method according to any one of claims 17 to 19, wherein the carbon dioxide gas is applied in a form of an external preparation for skin.
